Europäisches Patentamt

European Patent Office

Office européen des brevets

(11) Publication number: **0 048 737**
**B1**

# EUROPEAN PATENT SPECIFICATION

(45) Date of publication of patent specification: **13.02.85**

(51) Int. Cl.⁴: **A 61 M 27/00**

(21) Application number: **81901039.8**

(22) Date of filing: **05.03.81**

(86) International application number:
**PCT/US81/00287**

(87) International publication number:
**WO 81/02678 01.10.81 Gazette 81/23**

(54) **APPARATUS FOR DISPLACING FLUID IN TUBING.**

(30) Priority: **28.03.80 US 135062**

(43) Date of publication of application:
**07.04.82 Bulletin 82/14**

(45) Publication of the grant of the patent:
**13.02.85 Bulletin 85/07**

(84) Designated Contracting States:
**DE FR GB NL**

(56) References cited:
**DE-A-2 441 370**
**FR-A-1 440 769**
**FR-A-1 482 872**
**US-A-2 015 123**
**US-A-4 248 224**
**US-A-4 268 226**

(73) Proprietor: **SHERWOOD MEDICAL COMPANY**
**1831 Olive Street**
**St. Louis, MO 63103 (US)**

(72) Inventor: **BODICKY, Raymond, O.**
**5051 Lampglow Court**
**St. Louis, MO 63129 (US)**

(74) Representative: **Allen, William Guy Fairfax et al**
**J.A. KEMP & CO. 14 South Square Gray's Inn**
**London WC1R 5EU (GB)**

Courier Press, Leamington Spa, England.

## Description

This invention relates to the conveying of fluids through tubing and more particularly to manually operated fluid displacing means for fluid conveying tubing.

Tubing is used, for example, extensively in hospitals to convey fluids for various purposes including introducing fluids into the body and withdrawing fluids from the body. In body fluid drainage systems, for example, in chest drainage apparatus, an elastomeric tube is connected at one end to a chest drainage catheter connected to the pleural cavity of the patient, and the opposite end of the tube is connected to a fluid drainage collection bottle or chamber. Such drainage apparatus may collect drainage either by the effects of gravity alone or by a vacuum assist.

In such chest drainage systems, fibrin or a blood clot may occlude the tube or restrict the fluid discharge rate of flow. It has been common practice for the attendant to displace or clear the contents of the tube and remove the obstruction by hand. Because the tubing is generally made of a material which produces an outer surface with a relatively high coefficient of friction, such as polyvinyl chloride, latex, polyurethane or silicone, the fingers are generally first lubricated by applying an oil or grease to them. Then, while squeezing and compressing the tube between the fingers, the fingers are advanced longitudinally along the tube to move fluid and other matter through the tube thereby removing the obstruction or increasing the fluid flow rate. Clearing solids, semi-solids, or liquids from resilient, flexible tubing in various other fluid systems is often accomplished in a similar manner. If a lubricant is not employed, the friction between the fingers and tubing would cause chafing of the skin. Even when a lubricant is used, some chafing of the skin occurs and the tubing may not be cleared as well as it should be. Also, the use of lubricants is time consuming since it requires the application, as well as, removal of lubricant from the hand.

In order to avoid the above mentioned problems associated with the above manner of stripping or clearing the contents of tubing, various hand tools having rollers have been proposed. The tubing is clamped between the rollers and then the rollers are moved along the tubing to move the tubing contents longitudinally. Devices of this general type are disclosed in U.S. Patent Numbers 3,194,452, 3,648,701, and 4,164,223, and in Switzerland Patentschrift Number 278,763, February 16, 1952. These roller devices require a number of parts that must be assembled including pivotally connected arms carrying opposed tube rollers etc. These devices are not only relatively expensive but are relatively large and generally cumbersome in use.

It is now proposed, according to the invention, to provide apparatus for conveying fluids comprising compressible elastomeric tubing adapted to be connected to a source of fluid, to convey the fluid to fluid receiving means, a flexible sleeve surrounding a portion of the length of the tubing and manually compressible to compress the tubing, a coefficient of friction between the inner surface of the sleeve and the outer surface of the tubing being such as to enable said sleeve to slide freely along the tube while the sleeve is being manually compressed to compress the tubing.

The coefficient of friction of the inner surface of the sleeve with respect to a standard surface is lower than that of the outer surface of the tubing and advantageously at least a portion of the outer surface of the sleeve has a higher coefficient of friction with respect to a standard surface than the inner surface thereof. This serves to increase the friction between the outer surface of the sleeve and the hand of a person using the sleeve.

In order that the present invention may more readily be understood, the following description is given, merely by way of example, reference being made to the accompanying drawings, in which:—

Figure 1 is a perspective view of an apparatus for conveying fluid and having a hand fluid displacing device in accordance with a preferred embodiment of the invention;

Figure 2 is a persepctive view of the fluid conveying apparatus of Figure 1 illustrating the method of manually displacing the contents of the tubing of Figure 1;

Figure 3 is a perspective view of a tube contents displacing member in accordance with a modified embodiment; and

Figure 4 is a perspective view of a tube contents displacing member in accordance with another modified embodiment of the invention.

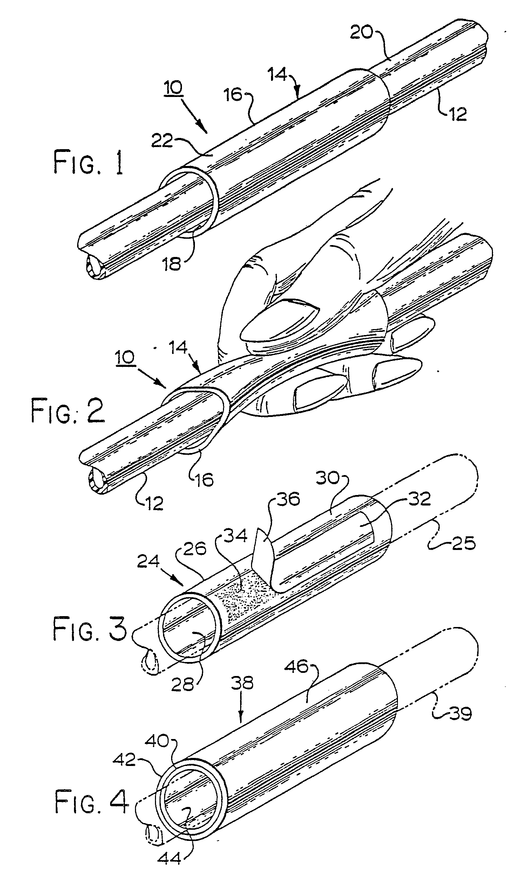

Referring now to the drawing and particularly to Figure 1, there is illustrated medical tubing apparatus, indicated generally at 10, including tubing 12 and a fluid displace or tube clearing member 14 on the tubing 12. Apparatus 10 is especially well suited for use in a chest drainage system. Tubing 12 may have one end connected to a catheter (not shown) that is connected to the pleural cavity of the patient. The opposite end of tubing 12 may be connected to a fluid drainage collection bottle or chamber (not shown). Drainage flow from the patient to the collection chamber may be effected by gravity or assisted by a suitable vacuum applied to the collection chamber.

The tubing 12 may be of any suitable rubber or plastic, for example, it may formed or extruded from a material which includes latex, polyvinyl chloride, silicone, polyurethane, latex or the like. The tubing 12 is an elastomeric tube which is flexible, resilient, and elastic, and which is collapsible or compressible, and returns to its original shape after deformation. Preferably, the opposed walls can be totally collapsed to effect a closure across the inside of the tube by manually pinching the tubing. Latex and polyvinyl chloride tubing is often used in chest drainage systems. All of the above material can be extruded to produce tubes, such as tubing 12. Such tubing has an outer surface which has a relatively high coefficient of friction with respect to the human skin such as on the fingers of the hand.

The fluid displacing or tube clearing member 14 is shown including a sleeve 16 surrounding the tubing 12 and having an inner diameter slightly greater than the outer diameter of tubing 12. For example, the outer diameter of tubing 12 may be 16.5 mm (.65 inch) and the inner diameter of sleeve 16 19 mm (.75 inch). Sleeve 16 is formed of a material that produces an inner surface 18 which has a relatively low coefficient of friction with respect to the outer surface 20 of the tubing 12 so that even when pressed together with some force, the sleeve 16 is readily slideable relative to the tubing 12. The sleeve 16 may be made from a plastic and is preferably made from Teflon, that is either polytetra fluoroethylene (TFE) or polyfluoroethylenepropylene (FEP). TFE is generally more economical than FEP. Either may be extruded to produce tubing from which sleeves, such as sleeve 16 can be cut. Such sleeves have a slick, i.e. low friction inner surface 18, as well as a slick or low friction outer surface 22. The sleeve may be relatively thin, for exmaple, a wall thickness of 0.38 mm (.015 inch) will generally be suitable. Since sleeve 16 is to be grasped by the hand or fingers of the person or attendant operating the fluid drainage system, as will be discussed hereafter, a suitable longitudinal length for the sleeve is about 76.2 mm (3 inches).

In use, if a blockage or partial occlusion of tube 12 occurs due, for example, to chest drainage matter clogging the tube, or if it is otherwise desired to increase the fluid flow rate of the tube contents, the operator hand grasps sleeve 16, such as between the fingers as shown in Figure 2. With the fingers engaging the outer surface 22, the sleeve 16 is pinched to compress or collapse the sleeve and tube to either occlude or partially occlude the tube 12. While maintaining the tube compressed, the fingers and sleeve 16 are advanced longitudinally along the tube 12 thereby causing the contents of the tube to advance and to generally displace tube contents into the collection chamber. This can break up a blockage and open the tube lumen for the free flow of fluid.

Dislodging drainage matter may also be accomplished by first pinching the tube 12 with one hand to occlude it, and simultaneously compressing the sleeve and tube, as indicated above, and advancing the compressed sleeve in a directon away from the occlusion. The creates a reduced or negative pressure in the tube 12 between the pinched zone and the compressed sleeve zone. When the one hand releases the pinched area of the tube, the pressure differential created on opposite sides of the occluding matter can cause such matter to be released or dislodged.

Because the inner surface 18 of the fluid displacing sleeve 16 has a low coefficient of friction compared to the outer surface 20 of tubing 12, the sleeve readily slides along the tubing 12 while it and the tubing 12 are compressed by the fingers. Since the fingers and sleeve move together there is no chafing or wear of the skin of the operator during the relative movement of the sleeve and tube. Relatively high pinching pressures can be applied through the sleeve 16 by the fingers to collapse tubing 12 and yet there is no chafing of the fingers.

As shown in Figure 3, fluid conveying apparatus is shown including a cylindrical fluid displacing or tube clearing member 24 of modified construction surrounding fluid conveying tubing shown in phantom at 25. The tube clearing member 24 includes a collapsible cylindrical sleeve 26 such as a thin sleeve or tube of plastic having a lower coefficient of friction than that of the tubing 25. Preferably, the sleeve 26 is formed of Teflon, either TFE or FEP, so that the inner surface 28 of the sleeve will be relatively slick and readily slideable over tubing 25. The outer surface 30 of the sleeve in such case will also be relatively slick.

In some cases, it is desirable to increase the friction between the fingers and the outer surface of the sleeve 26 so that there is little or substantially no relative movement between the fingers and sleeve during use of the tube clearing device. This is especially important where the attendant is subject to having water or other materials on the hands which might cause the fingers to slip off of the sleeve during use. A friction tape 32 is illustrated in Figure 3 as being applied longitudinally along the outer surface 30 of the sleeve. The tape 32 may be of the conventional type which has a layer 34 coated on both sides with a non-drying adhesive, and a peel-back paper cover layer 36. The tape 32 may be applied and the cover layer 36 removed as indicated in Figure 3 to expose the outer adhesive of layer 34. A plurality of such tapes may be applied where desired. The adhesive surface prevents or reduces relative movement between the fingers and the sleeve during use.

In Figure 4 a fluid conveying system is shown including a modified tube clearing device 38 surrounding resilient, compressible tubing 39. The clearing device includes an inner cylindrical sleeve 40 of material having a low coefficient of friction, preferably of TFE or FEP, and a concentric outer cylindrical sleeve 42 of a material having a relatively higher coefficient of friction than sleeve 40. The inner surface 44 of sleeve 40 has a lower coefficient of friction than tubing 39 so that it readily slides along the tubing 39 while compressing the tubing. The outer surface 46 of outer sleeve 42 provides a higher coefficient of friction between the fingers of the operator and the clearing device 38 to prevent or reduce relative movement during use or prevent the fingers from slipping off the device.

The outer sleeve 42 may be formed, for example, of any of the previously mentioned materials useful in making the fluid conveying tubing. The outer sleeve 42 may be secured to the inner Teflon layer by passing the inner sleeve through an extruder die and extruding a thermoplastic material, for example, polyvinyl chloride, onto the outer surface of the inner sleeve. Another method is to form the outer sleeve 42 of a highly elastic material such as a thin latex sleeve and stretch it onto the inner sleeve 40. In the latter case the resilience of the latex holds it in place.

The tube clearing devices described herein are simple in construction, light weight, economical and need no special storage area. Since they can be readily packaged with and disposed on the drainage tubing, the tube clearing device is immediately available for use in clearing or reducing obstructions or displacing contents of the tubing. It is, of course, especially important in emergency situations to have a tube clearing device at hand.

Because of the relatively low cost of the disclosed tube clearing devices, they can be employed extensively in hospitals, and can be discarded with the disposable tubing as a disposable item.

While the tube clearing devices and sleeves illustrated in the drawings are circumferentially continuous and are shown circular in cross-section when in an uncompressed state, other shapes may be used.

When one material or surface is stated herein to have a lower or higher coefficient of friction than a second surface or material, it is intended that the coefficient of friction of the two members or surfaces are compared when they are subjected to like pressures and moving forces when engaged against a standard surface (for example a glass surface).

## Claims

1. Apparatus for conveying fluids comprising compressible elastomeric tubing (12, 25, 39) adapted to be connected to a source of fluid, to convey the fluid to fluid receiving means, characterised in that a flexible sleeve (16, 26, 40) surrounds a portion of the length of the tubing and is manually compressible to compress the tubing, the coefficient of friction between the inner surface (18, 28, 44) of the sleeve and the outer surface (20) of the tubing being such as to enable said sleeve to slide freely along the tube while the sleeve is being manually compressed to compress the tubing.

2. Apparatus according to claim 1, characterised in that the coefficient of friction of the inner surface of the sleeve with respect to a standard surface is lower than that of the outer surface of the tubing.

3. Apparatus according to claim 1 or 2, characterised in that at least a portion of the outer surface (22, 30, 46) of the sleeve has a higher coefficient of friction with respect to a standard surface than the inner surface (18, 28, 44) thereof.

4. Apparatus according to claim 3, characterised in that an adhesive layer (34) is applied to the outer surface (30) of the sleeve (26) to provide a hand gripping surface of higher coefficient of friction with respect to a standard surface than the inner surface of the sleeve.

5. Apparatus according to claim 4, characterised in that the adhesive layer (34) is a portion of an adhesive strip (32) having a peelable outer backing (36) thereon, which is removable to expose the adhesive layer.

6. Apparatus according to claim 3, characterised in that the sleeve comprises an inner sleeve (40) surrounded by an outer sleeve (42), the outer surface (46) of the outer sleeve having a higher coefficient of friction with respect to a standard surface than the inner surface (44) of the inner sleeve (40) in contact with the tubing (39).

7. Apparatus according to any preceding claim, characterised in that the sleeve has an axial length such as to enable it to be readily gripped by the thumb and several fingers of a user.

8. Apparatus according to any preceding claim, characterised in that the sleeve is formed of polytetrafluoroethylene or polyfluoroethylenepropylene.

9. Apparatus according to any preceding claim, characterised in that the tubing is made of latex, polyurethane, silicone or polyvinyl chloride.

10. Apparatus according to any preceding claim, characterised in that the sleeve is circumferentially continuous.

11. Apparatus according to any preceding claim, characterised in that the sleeve is normally circular in cross-section when uncompressed.

## Revendications

1. Appareil pour déplacer des fluides, comprenant un tuyau compressible en élastomère (1, 25, 39) agencé pour être relié à une source de fluides, pour déplacer le fluide jusqu'à des moyens de réception de fluide, caractérisé par le fait qu'un manchon flexible (16, 26, 40) entoure une partie de la longueur du tuyau et peut être comprimé manuellement pour comprimer le tuyau, le coefficient de frottement entre la surface intérieure (18, 28, 44) du manchon et la surface extérieure (20) du tuyau étant tel qu'il permet audit manchon de glisser librement le long du tuyau alors que le manchon est comprimé manuellement pour comprimer le tuyau.

2. Appareil selon la revendication 1, caractérisé par le fait que le coefficient de frottement de la surface intérieure du manchon par rapport à une surface de référence est inférieur à celui de la surface extérieure du tuyau.

3. Appareil selon la revendication 1 ou 2, caractérisé par le fait qu'au moins une partie de la surface extérieure (22, 30, 46) du tuyau a un coefficient de frottement par rapport à une surface de référence supérieur à sa surface intérieure (18, 28, 44).

4. Appareil selon la revendication 3, caractérisé par le fait qu'une couche adhésive (34) est appliquée à la surface extérieure (30) du manchon (26) pour fournir une surface d'accrochage pour la main de coefficient de frottement par rapport à une surface de référence supérieur à celui de la surface intérieure du manchon.

5. Appareil selon la revendication 4, caractérisé par le fait que la couche adhésive (34) est une partie d'une bande adhésive (32) possédant un revêtement extérieur arrachable (36) qui peut être enlevé pour exposer la couche adhésive.

6. Appareil selon la revendication 3, caractérisé par le fait que le manchon comprend un manchon intérieur (40) entouré par un manchon extérieur (42), la surface extérieure (46) du manchon extérieur possédant un coefficient de frottement par rapport à une surface de référence supérieur à celui de la surface intérieure (44) du manchon intérieur (40) en contact avec le tuyau (39).

7. Appareil selon l'une quelconque des revendications précédentes, caractérisé par le fait que le manchon possède une longueur axiale telle qu'elle lui permet d'être facilement aggripé par le pouce et plusieurs doigts d'un utilisateur.

8. Appareil selon l'une quelconque des revendications précédentes, caractérisé par le fait que le manchon est réalisé en polytétrafluoroéthylène ou en polyfluoroéthylènepropylène.

9. Appareil selon l'une quelconque des revendications précédentes, caractérisé par le fait que le tuyau est réalisé en latex, polyuréthane, silicone ou chlorure de polyvinyle.

10. Appareil selon l'une quelconque des revendications précédentes, caractérisé par le fait que le manchon est continu circonférentiellement.

11. Appareil selon l'une quelconque des revendications précédentes, caractérisé par le fait que le manchon est normalement circulaire en coupe lorsqu'il n'est pas comprimé.

## Patentansprüche

1. Vorrichtung zur Förderung von Flüssigkeiten mit einer an eine Flüssigkeitsquelle anzuschließenden, die Flüssigkeit zu einer Flüssigkeitsaufnahmeeinrichtung fördernden zusammendrückbaren elastomeren Röhre (12, 25, 39), dadurch gekennzeichnet, daß eine flexible Manschette (16, 26, 40) einen Teil der Länge der Röhre umschließt sowie zum Zusammendrücken der Röhre von Hand zusammenpreßbar ist und daß der Reibungskoeffizient zwischen der Innenoberfläche (18, 28, 44) der Manschette und der Außenoberfläche (20) der Röhre derart ist, daß die Manschette frei längs der Röhre gleiten kann, während die Manschette zum Zusammendrücken der Röhre von hand zusammengepreßt wird.

2. Vorrichtung nach Anspruch 1, dadurch gekennzeichnet, daß der Reibungskoeffizient der Innenoberfläche der Manschette mit Bezug zu einer Standard-Oberfläche geringer ist als derjenige der Außenoberfläche der Röhre.

3. Vorrichtung nach Anspruch 1 oder 2, dadurch gekennzeichnet, daß wenigstens ein Teil der Außenoberfläche (22, 30, 46) der Manschette einen mit Bezug zu einer Standard-Oberfläche höheren Reibungskoeffizienten hat als die Innenoberfläche (18, 28, 44) der Manschette.

4. Vorrichtung nach Anspruch 3, dadurch gekennzeichnet, daß an der Außenoberfläche (30) der Manschette (26) eine haftfähige Schicht (34) angebracht ist, die eine Grifffläche mit einem Bezug zu einer Standard-Oberfläche höheren Reibungskoeffizienten als die Innenoberfläche der Manschette bildet.

5. Vorrichtung nach Anspruch 4, dadurch gekennzeichnet, daß die haftfähige Schicht (34) ein Teil eines Kleibestreifens (32) ist, der eine abziehbare, zum Freilegen der haftfähigen Schicht entfernbare äußere Abdecklage aufweist.

6. Vorrichtung nach Anspruch 3, dadurch gekennzeichnet, daß die Manschette eine von einem äußeren Mantelrohr (42) umgebene innere Hülse (40) aufweist und daß die Außenoberfläche (46) des äußeren Mantelrohres mit Bezug zu einer Standard-Oberfläche einen höheren Reibungskoeffizienten hat als die mit der Röhre (39) in Berührung befindliche Innenoberfläche (44) der inneren Hülse (40).

7. Vorrichtung nach einem der vorhergehenden Ansprüche, dadurch gekennzeichnet, daß die Manschette eine derartige axiale Länge hat, daß sie leicht vom Daumen und mehreren Fingern eines Benutzers zu ergreifen ist.

8. Vorrichtung nach einem der vorhergehenden Ansprüche, dadurch gekennzeichnet, daß die Manschette aus Polytetrafluoräthylen oder Polyfluoräthylenpropylen gebildet ist.

9. Vorrichtung nach einem der vorhergehenden Ansprüche, dadurch gekennzeichnet, daß die Röhre aus Latex, Polyurethan, Silikon oder Polyvinylchlorid gebildet ist.

10. Vorrichtung nach einem der vorhergehenden Ansprüche, dadurch gekennzeichnet, daß die Manschette umfangsseitig ununterbrochen ist.

11. Vorrichtung nach einem der vorhergehenden Ansprüche, dadurch gekennzeichnet, daß die Manschette im nichtzusammengepreßten Zustand normalerweise einen kreisförmigen Querschnitt hat.

FIG. 1

FIG. 2

FIG. 3

FIG. 4